# EUROPÄISCHE PATENTSCHRIFT

(11) **EP 3 723 864 B1**
(45) Veröffentlichungstag und Bekanntmachung des Hinweises auf die Patenterteilung: **14.01.2026**
(21) Anmeldenummer: 18792877.5
(22) Anmeldetag: 16.10.2018
(51) Int. Cl.: A61Q 5/06, A61K 8/81

(54) **VERDICKTES EIN-KOMPONENTEN-HAARTÖNUNGSMITTEL**
THICKENED ONE-COMPONENT HAIR TONER
AGENT DE COLORATION CAPILLAIRE ÉPAISSI À UN COMPOSANT

(30) Priorität: 15.12.2017 DE 102017222955
(43) Veröffentlichungstag der Anmeldung: 21.10.2020
(73) Patentinhaber: Henkel AG & Co. KGaA, 40589 Düsseldorf (DE)
(72) Erfinder: SCHOEPGENS, Juergen, 41366 Schwalmtal (DE); LECHNER, Torsten, 40764 Langenfeld (DE)
(86) Internationale Anmeldenummer: PCT/EP2018/078134
(87) Internationale Veröffentlichungsnummer: WO 2019/115058

(56) Entgegenhaltungen:
- EP-A1- 1 669 107
- WO-A1-2017/124061
- WO-A1-99/13822
- WO-A2-2009/077302
- US-A- 3 215 604
- US-A1- 2009 297 644
- US-A1- 2014 336 308

## Beschreibung

Die vorliegende Anmeldung betrifft ein Mittel zum Färben von keratinischen Fasern, insbesondere menschlichen Haaren, und ein Verfahren zur nicht-oxidativen, vorzugsweise semipermanenten, Färbung von keratinischen Fasern, insbesondere menschlichen Haaren.

Die Veränderung von Form und Farbe von keratinischen Fasern, insbesondere von Haaren, stellt einen wichtigen Bereich der modernen Kosmetik dar. Hierdurch kann das Erscheinungsbild der Haare sowohl aktuellen Modeströmungen als auch den individuellen Wünschen der einzelnen Person angepasst werden. Zur Veränderung der Haarfarbe kennt der Fachmann je nach Anforderung an die Färbung diverse Färbesysteme. Für permanente, intensive Färbungen mit guten Echtheitseigenschaften und guter Grauabdeckung werden üblicherweise Oxidationsfärbemittel verwendet. Solche Färbemittel enthalten üblicherweise Oxidationsfarbstoffvorprodukte, so genannte Entwicklerkomponenten und Kupplerkomponenten, die unter dem Einfluss von Oxidationsmitteln wie beispielsweise Wasserstoffperoxid untereinander die eigentlichen Farbstoffe ausbilden. Oxidationsfärbemittel zeichnen sich durch hervorragende, langanhaltende Färbeergebnisse aus, sind jedoch auch mit einem gewissen Ausmaß an Haarschädigung verbunden.

Möchte der Anwender Haarschädigungen reduzieren oder die Haarfarbe nur temporär verändern, kann er auf Färbemittel zurückgreifen, die auf direktziehenden Farbstoffen basieren. Hierbei diffundieren bereits fertig ausgebildete Farbstoffe aus dem Färbemittel in die Haarfaser hinein. Im Vergleich zur oxidativen Haarfärbung weisen die mit direktziehenden Farbstoffen erhaltenen Färbungen eine geringere Haltbarkeit und schnellere Auswaschbarkeit auf. Von Vorteil ist jedoch die geringere Haarschädigung der Färbung mit direktziehenden Farbstoffen.

Die vorgenannten direktziehenden Farbstoffe sind üblicherweise in einen kosmetisch geeigneten Träger, wie beispielsweise eine Creme, eingearbeitet. Der Träger gewährleistet eine homogene Verteilung und eine ausreichende Verweilzeit des Haarfärbemittels auf dem Haar.

Nachteilig ist die aufwändige Herstellung einer solchen Creme. Für das Aufschmelzen der Fettkomponenten und die Emulgierung wird viel Energie benötigt. Das anschließende Abkühlen verbraucht große Mengen an Kühlwasser.

Aufgrund von hohen Fettanteilen in herkömmlichen Cremes ist eine kontinuierliche Produktion nur schwierig zu realisieren. Hierbei müssen die Bestandteile in der Schmelze zueinander gegeben werden, um eine Pumpbarkeit zu gewährleisten.

US2014/0336308A1 beschreibt Mittel zum Schminken der Haut und der Lippen, die vorgeliertes Natriumpolyacrylat und einen Direktfarbstoff enthalten und sich in einem Kaltprozess herstellen lassen.

Der vorliegenden Erfindung lag die Aufgabe zu Grunde, ein nicht-oxidatives Mittel zum nicht-permanenten Färben von keratinischen Fasern, insbesondere menschlichen Haaren, bereitzustellen, das sich unter möglichst ökonomischen und nachhaltigen Bedingungen herstellen lässt. Weiterhin lag der vorliegenden Erfindung die Aufgabe zu Grunde, ein Mittel zum nicht-permanenten Färben von keratinischen Fasern, insbesondere menschlichen Haaren, bereitzustellen, das sich unter möglichst ökonomischen und nachhaltigen Bedingungen verpacken lässt. Weiterhin lag der vorliegenden Erfindung die Aufgabe zu Grunde, ein Mittel zum nicht-permanenten Färben von keratinischen Fasern, insbesondere menschlichen Haaren, bereitzustellen, das kontinuierlich herstellbar ist.

Weiterhin soll sich das nicht-oxidative Mittel zum nicht-permanenten Färben von keratinischen Fasern, insbesondere menschlichen Haaren, durch eine gute Haltbarkeit an den keratinischen Fasern für ein nicht oxidatives, nicht-permanentes Färbemittel auch nach mehreren Haarwäschen, eine einfache Applikation und im Allgemeinen kurze Einwirkzeiten auszeichnen, und zu guten optischen Ergebnissen, z.B. in Bezug auf die Färbeleistung und Echtheitseigenschaften, führen.

Gelöst werden diese Aufgaben durch nicht-oxidative Mittel zum nicht-permanenten Färben von keratinischen Fasern, insbesondere menschlichen Haaren, enthaltend in einem kosmetischen Träger
(a) mindestens einen direktziehenden Farbstoff in einer Gesamtmenge von 0,01 - 5 Gew.-%, bezogen auf das Gesamtgewicht des Mittels,
(b) Natriumpolyacrylat mit der CAS-Nummer 9003-04-7mit einer massenmittleren Molmasse M_{w} im Bereich von 1.000.000 bis 20.000.000 Dalton, bevorzugt 6.000.000 bis 15.000.000 Dalton, in einer Gesamtmenge von 0,6 - 3 Gew.-%, bezogen auf das Gewicht des Mittels,
(c) 70 - 95 Gew.-% Wasser, bezogen auf das Gewicht des Färbemittels,

dadurch gekennzeichnet, dass weiterhin mindestens ein vernetztes Copolymer aus Acrylsäure und nicht-ethoxylierten Estern der Acrylsäure mit linearen C10-C30-Monoalkoholen (d) enthalten ist, wobei das vernetzte Copolymer (d) ausgewählt ist aus Copolymeren mit der INCI-Bezeichnung Acrylates/C10-30 Alkyl Acrylate Crosspolymer,
wobei das nicht-oxidative Färbemittel dadurch erhalten wird, dass das Natriumpolyacrylat als in einer Wasser-in-Öl-Emulsion vorgeliert eingesetzt wird, wobei besagte Wasser-in-Öl-Emulsion, jeweils bezogen auf ihr Gewicht, 40 - 60 Gew.-% Natriumpolyacrylat, insgesamt 25 - 45 Gew.-% Öl(e), insgesamt 0,5 - 4,9 Gew.-% Tensid(e), darunter 0,5 - 4,9 Gew.-% Niotensid(e), und 0,5 - 4,9 Gew.-% Wasser enthält.

Unter keratinischen Fasern, keratinhaltigen Fasern oder Keratinfasern sind Pelze, Wolle, Federn und insbesondere menschliche Haare zu verstehen. Obwohl die erfindungsgemäßen Mittel in erster Linie zum Aufhellen und Färben von Keratinfasern geeignet sind, steht prinzipiell einer Verwendung auch auf anderen Gebieten nichts entgegen.

Kennzeichnend für das erfindungsgemäße Mittel ist die Anwesenheit von Natriumpolyacrylat mit der CAS-Nummer 9003-04-7 und mit einer massenmittleren Molmasse M_{w} im Bereich von 1.000.000 bis 20.000.000 Dalton, bevorzugt 6.000.000 bis 15.000.000 Dalton in einer Gesamtmenge von 0,6 - 3 Gew.-%, bezogen auf das Gewicht des Mittels, neben dem direktziehenden Farbstoff. Mit dem erfindungsgemäßen Mittel werden die vorstehend genannten Aufgaben gelöst, insbesondere in erfindungsgemäßen Mitteln in Form einer Creme, einer Emulsion oder eines Gels.

Alle Angaben zu Aggregatzuständen in der vorliegenden Anmeldung beziehen sich, soweit nicht anders angegeben, auf eine Temperatur von 20°C und einen Druck von 1013 mbar.

### (a) Mindestens ein direktziehender Farbstoff

Bei direktziehenden Farbstoffen handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole.

Direktziehende Farbstoffe können in anionische, kationische und nichtionische direktziehende Farbstoffe unterteilt werden.

Die Begriffe anionischer Farbstoff und Säurefarbstoff werden im Sinne dieser Erfindung synonym verwendet. Unter anionischen Farbstoffen bzw. Säurefarbstoffen werden direktziehende Farbstoffe verstanden, die mindestens eine Carbonsäuregruppierung (-COOH) und/oder mindestens eine Sulfonsäuregruppierung (-SO3H) besitzen. In Abhängigkeit vom pH-Wert liegen die protonierten Formen (-COOH, -SO3H) der Carbonsäure- bzw. Sulfonsäuregruppierungen mit ihren deprotonierten Formen (-COO<->, -SO3<-> vor) im Gleichgewicht vor. Mit abnehmendem pH-Wert steigt der Anteil der protonierten Formen. Werden direktziehende Farbstoffe in Form ihrer Salze eingesetzt, so liegen die Carbonsäuregruppen bzw. Sulfonsäuregruppen in deprotonierter Form vor und sind zur Einhaltung der Elektroneutralität mit entsprechenden stöchiometrischen Äquivalenten an Kationen (wie beispielsweise Na-Kation oder K-Kationen) neutralisiert. Ein anionischer Farbstoff trägt keine kationischen Ladungen.

Farbstoffe, die ausschließlich kationische Ladungen tragen, werden üblicherweise auch als basische Farbstoffe bezeichnet. Bei Farbstoffen, die ausschließlich anionische Ladungen tragen, spricht der Fachmann von Säurefarbstoffen.

Geeignete Säurefarbstoffe bzw. anionische direktziehende Farbstoffe sind ausgewählt aus Tetrabromphenolblau (CAS-Nr. 4430-25-5), Acid Yellow 1 (D&C Yellow 7, Citronin A, Ext. D&C Yellow No. 7, Japan Yellow 403 CI 10316, COLIPA n° B001), Acid Yellow 3 (COLIPA n°: C 54, D&C Yellow N° 10, Quinoline Yellow, E104, Food Yellow 13), Acid Yellow 9 (CI 13015), Acid Yellow 17 (CI 18965), Acid Yellow 23 (COLIPA n° C 29, Covacap Jaune W 1100 (LCW), Sicovit Tartrazine 85 E 102 (BASF), Tartrazine, Food Yellow 4, Japan Yellow 4, FD&C Yellow No. 5), Acid Yellow 36 (CI 13065), Acid Yellow 121 (CI 18690), Acid Orange 6 (CI 14270), Acid Orange 7 (2-Naphthol orange, Orange II, CI 15510, D&C Orange 4, COLIPA n° C015), Acid Orange 10 (C.I. 16230; Orange G sodium salt), Acid Orange 11 (CI 45370), Acid Orange 15 (CI 50120), Acid Orange 20 (CI 14600), Acid Orange 24 (Brown 1; CI 20170; KATSU201; Brown No.201; Resorcin Brown; Acid Orange 24; Japan Brown 201; D & C Brown No.1), Acid Red 14 (C.I.14720), Acid Red 18 (E124, Red 18; CI 16255), Acid Red 27 (E123, CI 16185, C-Rot 46, Echtrot D, FD&C Red Nr.2, Food Red 9, Naphtholrot S), Acid Red 33 (Red 33, Fuchsia Red, D&C Red 33, CI 17200), Acid Red 35 (C.I.18065), Acid Red 51 (CI 45430, Pyrosin B, Tetraiodfluorescein, Eosin J, lodeosin), Acid Red 52 (CI 45100, Food Red 106, Solar Rhodamine B, Acid Rhodamine B, Red n° 106 Pontacyl Brilliant Pink), Acid Red 73 (CI 27290), Acid Red 87 (Eosin, CI 45380), Acid Red 95 (CI 45425, Erythrosine, Simacid Erythrosine Y), Acid Red 184 (CI 15685), Acid Red 195, Acid Violet 43 (Jarocol Violet 43, Ext. D&C Violet n° 2, C.I. 60730, COLIPA n° C063), Acid Violet 49 (CI 42640), Acid Violet 50 (CI 50325), Acid Blue 1 (Patent Blue, CI 42045), Acid Blue 3 (Patent Blau V, CI 42051), Acid Blue 7 (CI 42080), Acid Blue 104 (CI 42735), Acid Blue 9 (E 133, Patentblau AE, Amidoblau AE, Erioglaucin A, CI 42090, C.I. Food Blue 2), Acid Blue 62 (CI 62045), Acid Blue 74 (E 132, CI 73015), Acid Blue 80 (CI 61585), Acid Green 3 (CI 42085, Foodgreen1), Acid Green 5 (CI 42095), Acid Green 9 (C.I. 42100), Acid Green 22 (C.I. 42170), Acid Green 25 (CI 61570, Japan Green 201, D&C Green No. 5), Acid Green 50 (Brillantsäuregrün BS, C.I. 44090, Acid Brilliant Green BS, E 142), Acid Black 1 (Black n° 401, Naphthalene Black 10B, Amido Black 10B, CI 20 470, COLIPA n° B15), Acid Black 52 (CI 15711), Food Yellow 8 (CI 14270), Food Blue 5, D&C Yellow 8, D&C Green 5, D&C Orange 10, D&C Orange 11, D&C Red 21, D&C Red 27, D&C Red 33, D&C Violet 2 und D&C Brown 1 sowie Mischungen dieser Farbstoffe. Bevorzugte anionische direktziehende Farbstoffe sind die unter den Bezeichnungen Acid Yellow 1, Acid Yellow 3, Acid Yellow 9, Acid Yellow 17, Acid Yellow 23, Acid Yellow 36, Acid Yellow 121, Acid Orange 6, Acid Orange 7, Acid Orange 10, Acid Orange 11, Acid Orange 15, Acid Orange 20, Acid Orange 24, Acid Red 14, Acid Red 18, Acid Red 27, Acid Red 33, Acid Red 35, Acid Red 51, Acid Red 52, Acid Red 73, Acid Red 87, Acid Red 95, Acid Red 184, Acid Red 195, Acid Violet 43, Acid Violet 49, Acid Violet 50, Acid Blue 1, Acid Blue 3, Acid Blue 7, Acid Blue 104, Acid Blue 9, Acid Blue 62, Acid Blue 74, Acid Blue 80, Acid Green 3, Acid Green 5, Acid Green 9, Acid Green 22, Acid Green 25, Acid Green 50, Acid Black 1, Acid Black 52, Food Yellow 8, Food Blue 5, D&C Yellow 7, D&C Yellow 8, D&C Orange 4, D&C Green 5, D&C Orange 10, D&C Orange 11, D&C Red 21, D&C Red 27, D&C Red 33, D&C Violet 2 und/oder D&C Brown 1 und Tetrabromphenolblau bekannten Verbindungen sowie Mischungen dieser Farbstoffe.

Bevorzugte kationische direktziehende Farbstoffe sind kationische Triphenylmethanfarbstoffe, wie Basic Blue 7, Basic Blue 26, Basic Violet 2 und Basic Violet 14 sowie aromatische Systeme, die mit einer quaternären Stickstoffgruppe substituiert sind, wie Basic Yellow 57, Basic Red 76, Basic Blue 99, Basic Brown 16 und Basic Brown 17 und HC Blue 16, sowie Basic Yellow 87, Basic Orange 31 und Basic Red 51.

Bevorzugte nichtionische direktziehende Farbstoffe sind ausgewählt aus HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9, sowie 1,4-Diamino-2-nitrobenzol, 2-Amino-4-nitrophenol, 1,4-Bis-(2-hydroxyethyl)amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol sowie Mischungen hiervon.

Weiterhin können als direktziehende Farbstoffe auch in der Natur vorkommende Farbstoffe eingesetzt werden, wie sie beispielsweise in Henna rot, Henna neutral, Henna schwarz, Kamillenblüte, Sandelholz, schwarzem Tee, Walnuss, Faulbaumrinde, Salbei, Blauholz, Krappwurzel, Catechu und Alkannawurzel enthalten sind.

Das erfindungsgemäße Mittel enthält mindestens einen direktziehenden Farbstoff in einer Gesamtmenge von 0,01 - 5 Gew.-%, bevorzugt von 0,1 - 4 Gew.-%, insbesondere von 0,2 - 3,5 Gew.-%, außerordentlich bevorzugt 0,3 - 3 Gew.-%, weiter außerordentlich bevorzugt 0,5 - 2 Gew.-%, weiter außerordentlich bevorzugt 0,7 - 1,2 Gew.-%, jeweils bezogen auf das Gewicht des erfindungsgemäßen Mittels.

### (b) Natriumpolyacrylat

Ein wesentliches Merkmal der erfindungsgemäßen Mittel ist die Anwesenheit von Natriumpolyacrylat mit einer massenmittleren Molmasse M_{w} im Bereich von 1.000.000 bis 20.000.000 Dalton in einer Gesamtmenge von 0,6 - 3 Gew.-%, bezogen auf das Gewicht des Mittels, wobei das Natriumpolyacrylat in einer Wasser-in-Öl-Emulsion vorgeliert vorliegt. Unter Natriumpolyacrylat werden erfindungsgemäß Polymere mit der CAS-Nummer 9003-04-7 verstanden. Erfindungsgemäß bevorzugte Natriumpolyacrylate weisen eine massenmittlere Molmasse M_{w} im Bereich von 6.000.000 bis 15.000.000 Dalton auf. Das mittlere Molekulargewicht M_{w} kann durch Gelpermeationschromatographie (GPC) mit Polystyrol als internem Standard gemäß DIN 55672-3, Version 8/2007, bestimmt werden.

Das in einer Wasser-in-Öl-Emulsion vorgelierte Natriumpolyacrylat führt zu einer Verdickung des Mittels, wobei das Mittel gleichzeitig die Konsistenz eines cremigen Gels erhält.

Das Natriumpolyacrylat ist als in einer Wasser-in-Öl-Emulsion vorgeliertes Natriumpolyacrylat enthalten, wobei die Natriumpolyacrylat-haltige Wasser-in-Öl-Emulsion, jeweils bezogen auf ihr Gesamtgewicht (von 100 Gew.-%), 40 - 60 Gew.-% Natriumpolyacrylat, insgesamt 25 - 45 Gew.-% Öl(e), insgesamt 0,5 - 4,9 Gew.-% Tensid(e), darunter 0,5 - 4,9 Gew.-% Niotensid(e), und 0,5 - 4,9 Gew.-% Wasser enthält.

Besonders bevorzugt ist das in der Natriumpolyacrylat-haltigen Wasser-in-Öl-Emulsion enthaltene Öl ausgewählt aus natürlichen und synthetischen Kohlenwasserstoffen, besonders bevorzugt aus Mineralöl, Paraffinölen, C₁₈-C₃₀-Isoparaffinen, insbesondere Isoeicosan, Polyisobutenen und Polydecenen, C₈-C₁₆-Isoparaffinen, sowie 1,3-Di-(2-ethylhexyl)-cyclohexan; den Benzoesäureestern von linearen oder verzweigten C₈₋₂₂-Alkanolen; Triglyceriden von linearen oder verzweigten, gesättigten oder ungesättigten, gegebenenfalls hydroxylierten C₈₋₃₀-Fettsäuren, insbesondere natürlichen Ölen; den Dicarbonsäureestern von linearen oder verzweigten C₂-C₁₀-Alkanolen; den Estern der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können; den Anlagerungsprodukten von 1 bis 5 Propylenoxid-Einheiten an ein- oder mehrwertige C₈₋₂₂-Alkanole; den C₈-C₂₂-Fettalkoholestern einwertiger oder mehrwertiger C₂-C₇-Hydroxycarbonsäuren; den symmetrischen, unsymmetrischen oder cyclischen Estern der Kohlensäure mit C₃₋₂₂-Alkanolen, C₃₋₂₂-Alkandiolen oder C₃₋₂₂-Alkantriolen; den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen; Siliconölen sowie Mischungen der vorgenannten Substanzen. Ein erfindungsgemäß besonders bevorzugtes Öl ist Mineralöl.

Das in der Natriumpolyacrylat-haltigen Wasser-in-Öl-Emulsion enthaltene Tensid ist ausgewählt aus nichtionischen Tensiden. Besonders bevorzugt verwendete nichtionische Tenside sind ausgewählt sind aus mit 7 - 80 Mol Ethylenoxid pro Mol ethoxyliertem Rizinusöl, ethoxylierten C₈-C₂₄-Alkanolen mit 5 - 30 Mol Ethylenoxid pro Mol, ethoxylierten C₈-C₂₄-Carbonsäuren mit 5 - 30 Mol Ethylenoxid pro Mol, mit 4 - 50 Mol Ethylenoxid pro Mol ethoxylierten Sorbitanmonoestern von linearen gesättigten und ungesättigten C₁₂ - C₃₀-Carbonsäuren, die hydroxyliert sein können, insbesondere diejenigen von Myristinsäure, Palmitinsäure, Stearinsäure oder von Mischungen dieser Fettsäuren, Alkylmono- und -oligoglycosiden mit 8 bis 22 Kohlenstoffatomen im Alkylrest und deren ethoxylierten Analoga, sowie Mischungen der vorgenannten Substanzen.

Die ethoxylierten C₈-C₂₄-Alkanole haben die Formel R¹O(CH₂CH₂O)ₙH, wobei R¹ steht für einen linearen oder verzweigten Alkyl- und/oder Alkenylrest mit 8 - 24 Kohlenstoffatomen und n, die mittlere Anzahl der Ethylenoxid-Einheiten pro Molekül, für Zahlen von 5 - 30, bevorzugt 6 - 20, besonders bevorzugt 6 - 12 Mol Ethylenoxid an 1 Mol Alkanol, das bevorzugt ausgewählt ist aus Caprylalkohol, 2-Ethylhexylalkohol, Caprinalkohol, Laurylalkohol, Isotridecylalkohol, Tridecylalkohol, Myristylalkohol, Cetylalkohol, Palmitoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie aus deren technischen Mischungen. Auch Addukte von 10 - 100 Mol Ethylenoxid an technische Fettalkohole mit 12 - 18 Kohlenstoffatomen, wie beispielsweise Kokos-, Palm-, Palmkern- oder Talgfettalkohol, sind geeignet. Besonders bevorzugt sind Trideceth-6, Isotrideceth-6, Undeceth-6, Myreth-6, Laureth-10, Laureth-12, Laureth-15, Laureth-20, Laureth-30, Myreth-10, Myreth-12, Myreth-15, Myreth-20, Myreth-30, Ceteth-10, Ceteth-12, Ceteth-15, Ceteth-20, Ceteth-30, Steareth-10, Steareth-12, Steareth-15, Steareth-20, Steareth-30, Oleth-10, Oleth-12, Oleth-15, Oleth-20, Oleth-30, Ceteareth-10, Ceteareth-15, Ceteareth-12, Ceteareth-15, Ceteareth-20, Ceteareth-30 sowie Coceth-10, Coceth-12, Coceth-15, Coceth-20 und Coceth-30; außerordentlich bevorzugt sind Trideceth-6 und Isotrideceth-6 sowie Mischungen hiervon.

Die ethoxylierten C₈-C₃₀-Carbonsäuren haben die Formel R¹O(CH₂CH₂O)ₙH, wobei R¹O steht für einen linearen oder verzweigten gesättigten oder ungesättigten Acylrest mit 8 - 30 Kohlenstoffatomen und n, die mittlere Anzahl der Ethylenoxid-Einheiten pro Molekül, für Zahlen von 5 - 30, bevorzugt 6 - 20, besonders bevorzugt 6 - 12 Mol Ethylenoxid an 1 Mol C₈-C₃₀-Carbonsäure, die bevorzugt ausgewählt ist aus Caprylsäure, 2-Ethylhexansäure, Caprinsäure, Laurinsäure, Isotridecansäure, Myristinsäure, Cetylsäure, Palmitoleinsäure, Stearinsäure, Isostearinsäure, Ölsäure, Elaidinsäure, Petroselinsäure, Arachyinsäure, Gadoleinsäure, Behensäure, Erucasäure und Brassidinsäure sowie aus deren technischen Mischungen. Auch Addukte von 5 - 30, bevorzugt 6 - 20, besonders bevorzugt 6 - 12 Mol Ethylenoxid an technische Fettsäuren mit 12 - 18 Kohlenstoffatomen, wie Kokos-, Palm-, Palmkern- oder Talgfettsäure, sind geeignet.

Erfindungsgemäß außerordentlich bevorzugte Mittel sind dadurch gekennzeichnet, dass sie mindestens ein Natriumpolyacrylat mit einer massenmittleren Molmasse M_{w} im Bereich von 1.000.000 bis 20.000.000 Dalton, bevorzugt 6.000.000 bis 15.000.000 Dalton in einer Gesamtmenge von 0,6 - 3 Gew.-%, bezogen auf das Gewicht des Mittels, enthalten, wobei das Natriumpolyacrylat als in einer Wasser-in-Öl-Emulsion vorgeliert enthalten ist, wobei besagte Wasser-in-Öl-Emulsion, jeweils bezogen auf ihr Gesamtgewicht, 40 - 60 Gew.-% Natriumpolyacrylat, insgesamt 25 - 45 Gew.-% Mineralöl, insgesamt 0,5 - 4,9 Gew.-% Tensid(e), bevorzugt 0,5 - 4,9 Gew.-% Niotensid(e), und 0,5 - 4,9 Gew.-% Wasser enthält.

Bevorzugt enthält das erfindungsgemäße Mittel Natriumpolyacrylat in einer Gesamtmenge von 0,6 - 2 Gew.-%, bevorzugt von 0,6 - 1,1 Gew.-%, jeweils bezogen auf das Gewicht des Mittels.

### (c) Wassergehalt

Die erfindungsgemäßen nicht-oxidativen Färbemittel sind weiterhin durch einen Wassergehalt von 70 - 95 Gew.-%, vorzugsweise 78 - 91 Gew.-%, besonders bevorzugt 80 - 86 Gew.-%, jeweils bezogen auf das Gewicht des Färbemittels, gekennzeichnet.

Das erfindungsgemäße Mittel enthält des Weiteren mindestens ein vernetztes Copolymer aus Acrylsäure und nicht-ethoxylierten Estern der Acrylsäure mit linearen C10-C30-Monoalkoholen (d), wobei das vernetzte Copolymer (d) ausgewählt ist aus Copolymeren mit der INCI-Bezeichnung Acrylates/C 10-30 Alkyl Acrylate Crosspolymer.

### (d) Mindestens ein vernetztes Copolymer aus Acrylsäure und nicht-ethoxylierten Estern der Acrylsäure mit linearen C10-C30-Monoalkoholen

Die erfindungsgemäßen Mittel enthalten weiterhin mindestens ein vernetztes Copolymer, aufgebaut aus Acrylsäure und nicht-ethoxylierten Estern der Acrylsäure mit linearen C10-C30-Monoalkoholen als Monomeren, ausgewählt aus Copolymeren mit der INCI-Bezeichnung Acrylates/C10-30 Alkyl Acrylate Crosspolymer. Als Vernetzungsagenz ist bevorzugt Sucroseallylether oder Pentaerythritylallylether enthalten.

Erfindungsgemäß besonders bevorzugte vernetzte Copolymere aus Acrylsäure und nicht-ethoxylierten Estern der Acrylsäure mit linearen C10-C30-Monoalkoholen sind erhältlich durch Polymerisation einer Monomermischung, die - jeweils bezogen auf ihr Gewicht - 80 - 99 Gew.-%, bevorzugt 90 - 98 Gew. -%, Acrylsäure, mindestens einen nicht-ethoxylierten Ester der Acrylsäure mit linearen C10-C30-Monoalkoholen in einer Gesamtmenge von 0,9 - 19,9 Gew.-%, bevorzugt 2 - 10 Gew.-% sowie mindestens ein Vernetzungsagenz in einer Gesamtmenge von 0,1 - 4 Gew.-% enthält.

Weitere erfindungsgemäß besonders bevorzugte vernetzte Copolymere aus Acrylsäure und nicht-ethoxylierten Estern der Acrylsäure mit linearen C10-C30-Monoalkoholen sind dadurch gekennzeichnet, dass ihre 0,5 Gew.-%ige Dispersion in Wasser bei 25 °C und einem pH-Wert im Bereich von 5,8 - 6,3 eine Viskosität im Bereich von 45.000 bis 65.000 mPas aufweist, gemessen mit einem Brookfield RVF- oder Brookfield RVT-Viskosimeter bei einer Rotationsfrequenz von 20 min⁻¹ mit Spindel 7.

Bevorzugt enthält das erfindungsgemäße Mittel mindestens ein vernetztes Copolymer aus Acrylsäure und nicht-ethoxylierten Estern der Acrylsäure mit linearen C10-C30-Monoalkoholen in einer Gesamtmenge von 0,05 - 3 Gew.-%, vorzugsweise 0,1 - 1,5 Gew.-%, bevorzugt 0,2 - 1 Gew.-%, jeweils bezogen auf das Gewicht des Mittels.

### (e) Tenside

Vorzugsweise wird den erfindungsgemäßen Mitteln zusätzlich eine oberflächenaktive Substanz zugesetzt, wobei solche oberflächenaktive Substanzen je nach Anwendungsgebiet als Tenside oder als Emulgatoren bezeichnet werden: Sie sind bevorzugt aus anionischen, zwitterionischen, amphoteren und nichtionischen Tensiden und Emulgatoren ausgewählt.

Tenside und Emulgatoren im Sinne der vorliegenden Anmeldung sind amphiphile (bifunktionelle) Verbindungen, die aus mindestens einem hydrophoben und mindestens einem hydrophilen Molekülteil bestehen.

Der hydrophobe Rest ist bevorzugt eine Kohlenwasserstoffkette mit 8 bis 30 Kohlenstoff-Atomen, die gesättigt oder ungesättigt, linear oder verzweigt sein kann. Besonders bevorzugt ist diese C₈-C₃₀-Alkylkette linear. Basiseigenschaften der Tenside und Emulgatoren sind die orientierte Adsorption an Grenzflächen sowie die Aggregation zu Mizellen und die Ausbildung von lyotropen Phasen. Bei der Auswahl erfindungsgemäß geeigneter Tenside kann es bevorzugt sein, ein Gemisch von Tensiden einzusetzen, um die Eigenschaften der erfindungsgemäßen Mittel optimal einzustellen.

Als anionische Tenside eignen sich für die erfindungsgemäßen Mittel alle für die Verwendung am menschlichen Körper geeigneten anionischen oberflächenaktiven Stoffe, die eine wasserlöslich machende, anionische Gruppe, beispielsweise eine Sulfat-, Sulfonat- oder Phosphat-Gruppe, und eine lipophile Alkylgruppe mit etwa 8 bis 30 C-Atomen, bevorzugt 8 bis 24 C-Atomen im Molekül aufweisen, mit Ausnahme von linearen und verzweigten Fettsäuren mit 8 bis 30 C-Atomen und deren Salzen (Seifen). Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe, polyethoxylierte Ethercarbonsäuren, Acylsarcoside, Acyltauride, Acylisethionate, Sulfobernsteinsäuremono- und -dialkylester und Sulfobernsteinsäuremono-alkylpolyoxyethylester mit 1 bis 6 Ethylenoxidgruppen, lineare Alkansulfonate, lineare alpha-Olefinsulfonate, Sulfonate ungesättigter Fettsäuren mit bis zu 6 Doppelbindungen, alpha-Sulfofettsäuremethylester von Fettsäuren, C₈-C₂₀-Alkylsulfate und C₈-C₂₀-Alkylethersulfate mit 1 bis 15 Oxyethylgruppen, Gemische oberflächenaktiver Hydroxysulfonate, sulfatierte Hydroxyalkylpolyethylen- und/oder Hydroxyalkylenpropylenglykolether, Ester von Weinsäure oder Zitronensäure mit ethoxylierten oder propoxylierten Fettalkoholen, gegebenenfalls polyethoxylierte Alkyl- und/oder Alkenyletherphosphate, sulfatierte Fettsäurealkylenglykolester, sowie Monoglyceridsulfate und Monoglyceridethersulfate. Bevorzugte anionische Tenside sind ausgewählt aus C₈-C₂₀-Alkylsulfaten, C₈-C₂₀-Alkylethersulfaten und C₈-C₂₀-Ethercarbonsäuren, jeweils mit 8 bis 20 C-Atomen in der Alkylgruppe und 0 bis 12 Ethylenoxidgruppen im Molekül. Besonders bevorzugt ist Natriumlaureth(2)-sulfat.

Als zwitterionische Tenside werden solche oberflächenaktiven Verbindungen bezeichnet, die eine lipophile Alkylgruppe mit etwa 8 bis 30 C-Atomen, bevorzugt 8 bis 24 C-Atomen und mindestens eine quartäre Ammoniumgruppe und mindestens eine Carboxylat-, Sulfonat- oder Sulfat-Gruppe tragen. Besonders geeignete zwitterionische Tenside sind die so genannten Betaine wie die N-Alkyl-N,N-dimethylammonium-glycinate, beispielsweise Kokosalkyldimethylammoniumglycinat, N-Acylaminopropyl-N,N-dimethylammoniumglycinate, beispielsweise Kokosacylaminopropyl-dimethylammoniumglycinat (mit der INCI-Bezeichnung Cocamidopropyl Betaine), und 2-Alkyl-3-carboxymethyl-3-hydroxyethyl-imidazoline mit jeweils 8 bis 18 C-Atomen in der Alkyl- oder Acylgruppe sowie Kokosacylaminoethylhydroxyethylcarboxymethylglycinat. Ein bevorzugtes zwitterionisches Tensid ist das unter der INCI-Bezeichnung Cocamidopropyl Betaine bekannte Kokosacylaminopropyl-dimethylammoniumglycinat.

Unter amphoteren Tensiden werden solche oberflächenaktiven Verbindungen verstanden, die eine C₈-C₃₀-Alkyl- oder -Acylgruppe und mindestens eine freie Aminogruppe und mindestens eine -COOH- oder -SO₃H-Gruppe im Molekül enthalten und zur Ausbildung innerer Salze befähigt sind. Beispiele für geeignete amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 30 C-Atomen in der Alkylgruppe. Besonders bevorzugte amphotere Tenside sind N-Kokosalkylaminopropionat, Kokosacylaminoethylaminopropionat und C₁₂-C₁₈-Acylsarcosin.

Erfindungsgemäß bevorzugte anionische oder zwitterionische Tenside sind ausgewählt aus C₈-C₂₀-Alkylsulfaten, C₈-C₂₀-Alkylethersulfaten und C₈-C₂₀-Ethercarbonsäuren, jeweils mit 8 bis 20 C-Atomen in der Alkylgruppe und 0 bis 12 Ethylenoxidgruppen im Molekül, wobei Natriumlaureth(2)-sulfat besonders bevorzugt ist, weiterhin aus Kokosacylaminopropyl-dimethylammoniumglycinat sowie aus Mischungen dieser Tenside.

Erfindungsgemäß außerordentlich bevorzugte Mittel enthalten mindestens ein anionisches oder zwitterionisches Tensid, ausgewählt aus C₈-C₂₀-Alkylsulfaten, C₈-C₂₀-Alkylethersulfaten und C₈-C₂₀-Ethercarbonsäuren, jeweils mit 8 bis 20 C-Atomen in der Alkylgruppe und 0 bis 12 Ethylenoxidgruppen im Molekül, wobei Natriumlaureth(2)sulfat besonders bevorzugt ist, weiterhin aus Kokosacylaminopropyl-dimethylammoniumglycinat sowie aus Mischungen dieser Tenside.

Wenn ein Tensid in den erfindungsgemäßen Mitteln vorliegt, enthalten die erfindungsgemäßen Mittel mindestens ein Tensid, bevorzugt mindestens ein Anion- oder Zwitteriontensid, in einer Gesamtmenge von 0,3 - 5 Gew.-%, vorzugsweise 0,5 - 4 Gew.-%, jeweils bezogen auf das Gewicht des Mittels.

Besonders bevorzugt weisen die erfindungsgemäßen Mittel eine Viskosität im Bereich von 8.000 - 100.000 mPas, vorzugsweise 10.000 - 80.000 mPas, bevorzugt 12.000 - 60.000 mPas, insbesondere 13.000 - 50.000 mPas, auf, jeweils gemessen bei 20°C mit einem Brookfield-Rotationsviskosimeter bei einer Rotationsfrequenz von 4 min⁻¹ mit Spindel 5.

Das erfindungsgemäße Mittel weist üblicherweise einen pH-Wert von 2,5 - 11, gemessen bei 22°C, auf.

Je nachdem, welcher Typ an direktziehenden Farbstoffen (nichtionisch, kationisch oder anionisch) im erfindungsgemäßen Mittel eingesetzt werden soll, wird der pH-Wert bevorzugt entweder sauer, neutral oder basisch eingestellt.

In einer bevorzugten Ausführungsform weist das erfindungsgemäße Mittel einen pH-Wert im Bereich von 3,0 - 11, besonders bevorzugt 4,5 - 10,5, besonders bevorzugt 5,5 - 10, insbesondere 6 - 9,5, auf, jeweils gemessen bei 22°C.

Von der vorliegenden Anmeldung sind auch erfindungsgemäße Mittel umfasst, die einen pH-Wert im Bereich von im Allgemeinen 3 - 11, vorzugsweise 3,5 - 9,5, bevorzugt 4 - 8, insbesondere 5 - 7,5 aufweisen, jeweils gemessen bei 22°C.

Die Messung des pH-Wertes kann beispielsweise mit einer Glaselektrode erfolgen, die üblicherweise in Form einer Einstabmesskette ausgeführt ist. Bei den pH-Werten der vorliegenden Erfindung handelt es sich um pH-Werte, die bei einer Temperatur von 22 °C gemessen wurden.

Zur Einstellung pH-Wertes können dem erfindungsgemäßen Mittel zusätzlich Alkalisierungsmittel oder Ansäuerungsmittel (e) zugegeben werden.

Geeignete Alkalisierungsmittel sind bevorzugt ausgewählt aus der Gruppe bestehend aus Ammoniak, Alkanolaminen, basischen Aminosäuren und anorganischen Alkalisierungsmitteln. Bevorzugte anorganische Alkalisierungsmittel sind Magnesiumcarbonat, Natriumhydroxid, Kaliumhydroxid, Natriumsilicat und Natriummetasilicat. Alkanolamine sind bevorzugt ausgewählt aus Monoethanolamin (insbesondere 2-Aminoethanol), 2-Amino-2-methylpropanol und Triethanolamin. Die als Alkalisierungsmittel einsetzbaren basischen Aminosäuren sind bevorzugt ausgewählt aus der Gruppe bestehend aus Arginin, Lysin, Ornithin und Histidin, besonders bevorzugt Arginin.

Zur Einstellung des pH-Werts verwendbare Ansäuerungsmittel sind organische Säuren, wie Citronensäure, Essigsäure, Ascorbinsäure, Benzoesäure, Milchsäure, Äpfelsäure und Maleinsäure, sowie mineralische Säuren, wie Salzsäure, Schwefelsäure oder Phosphorsäure.

Wenn ein Alkalisierungsmittel oder Ansäuerungsmittel in den erfindungsgemäßen Mitteln eingesetzt wird, enthält das erfindungsgemäße Mittel mindestens ein Alkalisierungsmittel oder mindestens ein Ansäuerungsmittel in einer Gesamtmenge von 0,05 - 3 Gew.-%, vorzugsweise 0,1 - 2 Gew.-%, bevorzugt 0,2 - 1,5 Gew.-%, jeweils bezogen auf das Gewicht des Mittels.

Ferner können die erfindungsgemäßen Mittel weitere Wirk-, Hilfs- und Zusatzstoffe enthalten.

Geeignete Wirk-, Hilfs- und Zusatzstoffe sind beispielsweise Öle; nichtionische Polymere, wie beispielsweise Vinylpyrrolidinon/Vinylacrylat-Copolymere, Polyvinylpyrrolidinon, Vinylpyrrolidinon/ Vinylacetat-Copolymere, Polyethylenglykole und Polysiloxane; zusätzliche Silikone, wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/oder Polyalkylarylsiloxane, insbesondere Polysiloxane mit organofunktionellen Gruppen, wie substituierten oder unsubstituierten Aminen (Amodimethicone), Carboxyl-, Alkoxy- und/oder Hydroxylgruppen (Dimethiconcopolyole), lineare PolysiloxanA)-PolyoxyalkylenB)-Blockcopolymere, gepfropften Silikonpolymere; kationische Polymere wie quaternisierte Celluloseether, Polysiloxane mit quaternären Gruppen, Dimethyldiallylammoniumchlorid-Polymere, Acrylamid-Dimethyldiallyl-ammoniumchlorid-Copolymere, mit Diethylsulfat quaternierte Dimethylamino-ethylmethacrylat-Vinylpyrrolidinon-Copolymere, Vinylpyrrolidinon-Imidazoliniummethochlorid-Copolymere und quaternierter Polyvinylalkohol; zwitterionische und amphotere Polymere; haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis sowie in Form ihrer Fettsäure-Kondensationsprodukte oder gegebenenfalls anionisch oder kationisch modifizierten Derivate; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Wachse, wie Bienenwachs und Montanwachs; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel, wie Latex, Styrol/PVP-und Styrol/Acrylamid-Copolymere; Perlglanzmittel, wie Ethylenglykolmono- und -distearat sowie PEG-3-distearat und Pigmente.

Erfindungsgemäß besonders bevorzugte Öle sind ausgewählt aus den Estern der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können. Dazu zählen Cetyl-2-ethylhexanoat, 2-Hexyldecylstearat, 2-Hexyldecyllaurat, Isodecylneopentanoat, Isononylisononanoat, 2-Ethylhexylpalmitat und 2-Ethylhexylstearat. Ebenfalls bevorzugt sind Isopropylmyristat, Isopropylpalmitat, Isopropylstearat, Isopropylisostearat, Isopropyloleat, Isooctylstearat, Isononylstearat, Isocetylstearat, Isononylisononanoat, Isotridecylisononanoat, Cetearylisononanoat, 2-Ethylhexyllaurat, 2-Ethylhexylisostearat, 2-Ethylhexylcocoat, 2-Octyldodecylpalmitat, Butyloctansäure-2-butyloctanoat, Diisotridecylacetat, n-Butylstearat, n-Hexyllaurat, n-Decyloleat, Oleyloleat, Oleylerucat, Erucyloleat, Erucylerucat, Ethylenglycoldioleat und Ethylenglycoldipalmitat.

Weitere erfindungsgemäß bevorzugte Öle sind ausgewählt aus natürlichen und synthetischen Kohlenwasserstoffen, besonders bevorzugt aus Mineralölen, Paraffinölen, C₁₈-C₃₀-Isoparaffinen, insbesondere Isoeicosan, Polyisobutene und Polydecene, die beispielsweise unter der Bezeichnung Emery^{®} 3004, 3006, 3010 oder unter der Bezeichnung Ethylflo^{®} von Albemarle oder Nexbase^{®} 2004G von Nestle erhältlich sind, weiterhin ausgewählt aus C₈-C₁₆-Isoparaffinen, insbesondere aus Isodecan, Isododecan, Isotetradecan und Isohexadecan sowie Mischungen hiervon, sowie 1,3-Di-(2-ethylhexyl)-cyclohexan.

Weitere erfindungsgemäß bevorzugte Öle sind ausgewählt aus den Benzoesäureestern von linearen oder verzweigten C8-22-Alkanolen. Besonders bevorzugt sind Benzoesäure-C12-C15-alkylester, Benzoesäureisostearylester, Ethylhexylbenzoat und Benzoesäureoctyldocecylester.

Weitere erfindungsgemäß bevorzugte Öle sind ausgewählt aus Fettalkoholen mit 6 - 30 Kohlenstoff-atomen, die bei 20°C flüssig sind und ungesättigt oder verzweigt und gesättigt oder verzweigt und ungesättigt sein können. Bevorzugte Alkoholöle sind Laurylalkohol, ein Hauptbestandteil des Kokosfettalkohols, weiterhin 2-Octyldodecanol, 2-Hexyldecanol, 2-Ethylhexylalkohol und Isostearylalkohol.

Weitere bevorzugte Öle sind ausgewählt aus Mischungen von Guerbetalkoholen und Guerbetalkoholestern, z.B. Mischungen von 2-Hexyldecanol und 2-Hexyldecyllaurat.

Weitere erfindungsgemäß bevorzugte kosmetische Öle sind ausgewählt aus den Triglyceriden (= Dreifachestern des Glycerins) von linearen oder verzweigten, gesättigten oder ungesättigten, gegebenenfalls hydroxylierten C8-30-Fettsäuren. Besonders bevorzugt kann die Verwendung natürlicher Öle, z.B. Amaranthsamenöl, Aprikosenkernöl, Arganöl, Avocadoöl, Babassuöl, Baumwollsaatöl, Borretschsamenöl, Camelinaöl, Distelöl, Erdnussöl, Granatapfelkernöl, Grapefruitsamenöl, Hanf-öl, Haselnussöl, Holundersamenöl, Johannesbeersamenöl, Jojobaöl, Leinöl, Macadamianussöl, Maiskeimöl, Mandelöl, Marulaöl, Nachtkerzenöl, Olivenöl, Palmöl, Palmkernöl, Paranussöl, Pekannussöl, Pfirsichkernöl Rapsöl, Rizinusöl, Sanddornfruchtfleischöl, Sanddornkernöl, Sesamöl, Sojaöl, Sonnenblumenöl, Traubenkernöl, Walnussöl, Wildrosenöl, Weizenkeimöl, und die flüssigen Anteile des Kokosöls und dergleichen sein. Bevorzugt sind aber auch synthetische Triglyceridöle, insbesondere Capric/ Caprylic Triglycerides, z. B. die Handelsprodukte Myritol^{®} 318, Myritol^{®} 331 (BASF) oder Miglyol^{®} 812 (Hüls) mit unverzweigten Fettsäureresten sowie Glyceryltriisostearin mit verzweigten Fettsäureresten.

Weitere erfindungsgemäß besonders bevorzugte kosmetische Öle sind ausgewählt aus den Dicarbonsäureestern von linearen oder verzweigten C2-C10-Alkanolen, insbesondere Diisopropyladipat, Di-n-butyladipat, Di-(2-ethylhexyl)adipat, Dioctyladipat, Diethyl-/Di-n-butyl/ Dioctylsebacat, Diisopropylsebacat, Dioctylmalat, Dioctylmaleat, Dicaprylylmaleat, Diisooctylsuccinat, Di-2-ethylhexylsucci-nat und Di-(2-hexyldecyl)-succinat.

Weitere erfindungsgemäß bevorzugte kosmetische Öle sind ausgewählt aus den Anlagerungsprodukten von 1 bis 5 Propylenoxid-Einheiten an ein- oder mehrwertige C8-22-Alkanole, wie Octanol, Decanol, Decandiol, Laurylalkohol, Myristylalkohol und Stearylalkohol, z. B. PPG-2-Myristylether und PPG-3-Myristylether.

Weitere erfindungsgemäß bevorzugte kosmetische Öle sind ausgewählt aus den Anlagerungsprodukten von mindestens 6 Ethylenoxid- und/oder Propylenoxid-Einheiten an ein- oder mehrwertige C3-22-Alkanole wie Glycerin, Butanol, Butandiol, Myristylalkohol und Stearylalkohol, die gewünschtenfalls verestert sein können, z. B. PPG-14-Butylether, PPG-9-Butylether, PPG-10-Butandiol, PPG-15-Stearylether und Glycereth-7-diisononanoat.

Weitere erfindungsgemäß bevorzugte kosmetische Öle sind ausgewählt aus den C8-C22-Fettalko-holestern einwertiger oder mehrwertiger C2-C7-Hydroxycarbonsäuren, insbesondere die Ester der Glycolsäure, Milchsäure, Äpfelsäure, Weinsäure, Citronensäure und Salicylsäure.

Weitere erfindungsgemäß bevorzugte kosmetische Öle sind ausgewählt aus den symmetrischen, unsymmetrischen oder cyclischen Estern der Kohlensäure mit C₃₋₂₂-Alkanolen, C₃₋₂₂-Alkandiolen oder C₃₋₂₂-Alkantriolen, z. B. Dicaprylylcarbonat oder die Ester gemäß der Lehre der DE 19756454 A1, insbesondere Glycerincarbonat.

Weitere kosmetische Öle, die erfindungsgemäß bevorzugt sein können, sind ausgewählt aus den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen.

Weitere kosmetische Öle, die erfindungsgemäß geeignet sind, sind ausgewählt aus den Siliconölen, zu denen z. B. Dialkyl- und Alkylarylsiloxane, wie beispielsweise Cyclopentasiloxan, Cyclohexasiloxan, Dimethylpolysiloxan und Methylphenylpolysiloxan, aber auch Hexamethyldisiloxan, Octamethyltrisiloxan und Decamethyltetrasiloxan zählen. Bevorzugt können flüchtige Siliconöle sein, die cyclisch sein können, wie z. B. Octamethylcyclotetrasiloxan, Decamethylcyclopentasiloxan und Dodecamethylcyclohexasiloxan sowie Mischungen hiervon, wie sie z. B. in den Handelsprodukten DC 244, 245, 344 und 345 von Dow Corning enthalten sind. Ebenfalls geeignet sind flüchtige lineare Siliconöle, insbesondere Hexamethyldisiloxan (L₂), Octamethyltrisiloxan (L₃), Decamethyltetrasiloxan (L₄) sowie beliebige Zweier- und Dreiermischungen aus L₂, L₃ und/ oder L₄, bevorzugt solche Mischungen, wie sie z. B. in den Handelsprodukten DC 2-1184, Dow Corning^{®} 200 (0,65 cSt) und Dow Corning^{®} 200 (1,5 cSt) von Dow Corning enthalten sind. Bevorzugte nichtflüchtige Siliconöle sind ausgewählt aus höhermolekularen linearen Dimethylpolysiloxanen, im Handel erhältlich z. B. unter der Bezeichnung Dow Corning^{®} 190, Dow Corning^{®} 200 Fluid mit kinematischen Viskositäten (25°C) im Bereich von 5 - 100 cSt, bevorzugt 5 - 50 cSt oder auch 5 - 10 cSt, und Dimethylpolysiloxan mit einer kinematischen Viskosität (25°C) von 350 cSt.

Es kann erfindungsgemäß außerordentlich bevorzugt sein, Mischungen der vorgenannten Öle einzusetzen.

Bevorzugte erfindungsgemäße Färbemittel sind dadurch gekennzeichnet, dass das kosmetische Öl ausgewählt ist aus natürlichen und synthetischen Kohlenwasserstoffen, besonders bevorzugt aus Paraffinölen, C₁₈-C₃₀-Isoparaffinen, insbesondere Isoeicosan, Polyisobutene und Polydecene, C₈-C₁₆-Isoparaffinen, sowie 1,3-Di-(2-ethylhexyl)-cyclohexan; den Benzoesäureestern von linearen oder verzweigten C₈₋₂₂-Alkanolen; Fettalkoholen mit 6 - 30 Kohlenstoffatomen, die bei 20 °C und 1013 mbar flüssig sind und ungesättigt oder verzweigt und gesättigt oder verzweigt und ungesättigt sein können, bevorzugt Laurylalkohol und 2-Octyldodecanol; Triglyceriden von linearen oder verzweigten, gesättigten oder ungesättigten, gegebenenfalls hydroxylierten C₈₋₃₀-Fettsäuren, insbesondere natürlichen Ölen; den Dicarbonsäureestern von linearen oder verzweigten C₂-C₁₀-Alkanolen; den Estern der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können; den Anlagerungsprodukten von 1 bis 5 Propylenoxid-Einheiten an ein- oder mehrwertige C₈₋₂₂-Alkanole; den Anlagerungsprodukten von mindestens 6 Ethylenoxid- und/oder Propylenoxid-Einheiten an ein- oder mehrwertige C₃₋₂₂-Alkanole; den C₈-C₂₂-Fettalkoholestern einwertiger oder mehrwertiger C₂-C₇-Hydroxycarbonsäuren; den symmetrischen, unsymmetrischen oder cyclischen Estern der Kohlensäure mit C₃₋₂₂-Alkanolen, C₃₋₂₂-Alkandiolen oder C₃₋₂₂-Alkantriolen; den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen; Siliconölen sowie Mischungen der vorgenannten Substanzen.

Weitere erfindungsgemäße bevorzugte nicht-oxidative Färbemittel sind dadurch gekennzeichnet, dass sie, jeweils bezogen auf das Gewicht des Färbemittels, mindestens ein Öl in einer Gesamtmenge von 0,1 - 20 Gew.-%, bevorzugt 0,5 - 10 Gew.-%, besonders bevorzugt 1 bis 6 Gew.-%, enthalten.

Weitere erfindungsgemäße bevorzugte nicht-oxidative Färbemittel sind dadurch gekennzeichnet, dass sie mindestens einen C₁₄-C₃₀-Fettalkohol, der bei 20°C und 1013 mbar fest ist, enthalten.

Bevorzugte C₁₄-C₃₀-Fettalkohole, die bei 20°C und 1013 mbar fest sind, sind ausgewählt aus Tetra-decan-1-ol (Tetradecylalkohol, Myristylalkohol), Hexadecan-1-ol (Hexadecylalkohol, Cetylalkohol), Octadecan-1-ol (Octadecylalkohol, Stearylalkohol), Arachylalkohol (Eicosan-1-ol), Heneicosylalkohol (Heneicosan-1-ol) und/oder Behenylalkohol (Docosan-1-ol) sowie aus Mischungen hiervon.

Weitere erfindungsgemäße bevorzugte nicht-oxidative Färbemittel sind dadurch gekennzeichnet, dass sie, jeweils bezogen auf das Gewicht des Färbemittels, mindestens einen C₁₄-C₃₀-Fettalkohol, der bei 20°C und 1013 mbar fest ist, in einer Gesamtmenge von 0,1 - 10 Gew.-%, bevorzugt 0,3 - 5 Gew.-%, besonders bevorzugt 0,5 bis 2 Gew.-%, enthalten.

Die Auswahl dieser weiteren Wirk-, Hilfs- und Zusatzstoffe wird der Fachmann gemäß der gewünschten Eigenschaften der Mittel treffen.

Die zusätzlich zu den Ölen und festen C₁₄-C₃₀-Fettalkoholen vorstehend genannten Wirk-, Hilfs- und Zusatzstoffe können, wenn sie in den erfindungsgemäßen Mitteln eingesetzt werden, bevorzugt in Mengen von jeweils 0,001 - 25 Gew.-%, insbesondere von 0,1 - 15 Gew.-%, bezogen auf das Gewicht des Färbemittels, enthalten sein.

Das erfindungsgemäße Mittel kann grundsätzlich in Form von Cremes, Emulsionen, Gelen oder tensidhaltigen schäumenden Lösungen, wie beispielsweise Shampoos, Schaumaerosolen, Schaumformulierungen oder anderen Zubereitungen, vorliegen, die für die Anwendung auf dem Haar geeignet sind.

Bevorzugt liegt das erfindungsgemäße Mittel in Form einer Creme, einer Emulsion oder eines Gels vor.

Das erfindungsgemäße Mittel zeichnet sich durch eine gute Haltbarkeit an keratinischen Fasern auch nach mehreren Wäschen, eine einfache Applikation und im Allgemeinen kurze Einwirkzeiten aus.

Im Allgemeinen beträgt die Haltbarkeit des Farbstoffs des erfindungsgemäßen Mittels auf den keratinischen Fasern, insbesondere menschlichen Haaren, 1 bis 24 Wäschen, vorzugsweise von 2 bis 14 Wäschen, bei 10 - 50°C, vorzugsweise bei 25 - 40°C für 5 Minuten bis 2 Stunden.

Ein weiterer Gegenstand der vorliegenden Anmeldung ist ein Verfahren zur nicht-oxidativen, vorzugsweise semipermanenten, Färbung von keratinischen Fasern, insbesondere menschlichen Haaren, wobei das erfindungsgemäße Mittel direkt in Form eines Einkomponentenmittels auf die keratinischen Fasern appliziert wird, wobei das Verfahren vorzugsweise mit einer Einwirkzeit auf den keratinischen Fasern von 5 Sekunden bis 45 Minuten, bevorzugt 1 bis 40 Minuten, besonders bevorzugt 5 bis 30 Minuten, außerordentlich bevorzugt 10 - 20 Minuten, bei Raumtemperatur und/oder bei 30 - 60°C, vorzugsweise bei 32 - 50°C, durchgeführt wird.

Der Begriff "Raumtemperatur" bezeichnet erfindungsgemäß die Temperatur in dem Raum, in dem eine Person üblicherweise ein Haarfärbemittel benutzt, also üblicherweise ein Badezimmer oder ein Friseursalon, in dem eine Temperatur im Bereich von 10 - 29 °C herrscht.

Das Einwirkenlassen des erfindungsgemäßen Mittels kann auch bei mindestens 30 °C, bevorzugt bei 30 - 60°C, besonders bevorzugt bei 32 - 50°C stattfinden, wenn das Haar beispielsweise mit einer Wärmehaube oder mit einem Wärmestrahler erwärmt wird.

Für das erfindungsgemäße und für erfindungsgemäß bevorzugte Färbeverfahren gilt mutatis mutandis das Vorstehend zu den erfindungsgemäßen und erfindungsgemäß bevorzugten nicht-oxidativen Färbemitteln Gesagte.

Die nachfolgenden Beispiele sollen den Gegenstand der vorliegenden Erfindung verdeutlichen, ohne ihn hierauf zu beschränken.

### Beispiele

**Tabelle 1: Erfindungsgemäßes Färbemittel A**

| **Inhaltsstoff** | **Einwaage (Gew.-%)** |
|---|---|
| Acrylates/C10-30 Alkyl Acrylate Crosspolymer | 0,40 |
| Paraffinum Liquidum (ex ¹⁾) | 0,74 |
| C11-13 Isoparaffin (ex ¹⁾) | 0,06 |
| Trideceth-6 (ex ¹⁾) | 0,1 |
| Natriumpolyacrylat (ex ¹⁾) | 1,00 |
| 2-Octyldodecanol | 4,00 |
| Kokosfettalkohol, C12-18 | 0,75 |
| Fettalkohol-EO sulfat-Na C12-14 2EO | 3,00 |
| Monoethanolamin (2-Aminoethan-1-ol) | 1,14 |
| **HC Red** 3²⁾ | 2,00 |
| Wasser | ad 100,00 |

| | |
|---|---|
| ¹⁾ Flocare^{®} DP/ES-502¹: Wasser-in-Öl-Emulsion aus Natriumpolyacrylat, Mineralöl, Trideceth-6, C11-13 Isoparaffin und Wasser ²⁾ nichtionischer direktziehender Farbstoff | |

**Tabelle 2: Erfindungsgemäßes Färbemittel B**

| **Inhaltsstoff** | **Einwaage (Gew.-%)** |
|---|---|
| Acrylates/C10-30 Alkyl Acrylate Crosspolymer | 0,40 |
| Natriumpolyacrylat | (Aktivgehalt) 1,00 |
| Mineralöl | 0,74 |
| Trideceth-6 | 0,10 |
| C11-13 Isoparaffin | 0,06 |
| 2-Octyldodecanol | 4,00 |
| Fettalkohol, C12-18 | 0,75 |
| Fettalkohol-EO sulfat-Na C12-14 2EO | 0,81 |
| Monoethanolamin (2-Aminoethan-1-ol) | 1,13 |
| **HC Red** 3²⁾ | 2,00 |
| Wasser | ad 100,00 |

| | |
|---|---|
| ²⁾ **nichtionischer** direktziehender Farbstoff | |

**Tabelle 3: Vergleichsfärbemittel C**

| **Inhaltsstoff** | **Einwaage (Gew.-%)** |
|---|---|
| Lorol^{®1)} | 4,00 |
| Lanette^{®} D ²⁾ | 6,00 |
| Propylparaben | 0,18 |
| Methylparaben | 0,40 |
| Ceteareth-20 ³⁾ | 0,90 |
| Mackam^{®} 2CSF-40CG ⁴⁾ | 1,80 |
| HC Blue 12 ⁵⁾ | 0,05 |
| 4-Amino-3-nitrophenol | 1,00 |
| Polyethylene Glycol MG400 | 5,00 |
| Polyquaternium-6 | 0,50 |
| Nicotinamide | 0,15 |
| D-Panthenol 75% | 0,20 |
| Parfum | 0,30 |
| Wasser | ad 100,00 |

| | |
|---|---|
| ¹⁾ **Fettalkohol,** C12-18 ²⁾ Cetearyl Alkohol ³⁾ Polyethylenglycolether des Cetearylakohols (durchschnittlich 20 Einheiten -CH2-CH2-O-) ⁴⁾ INCI Name: Disodium Cocoamphodipropionate ⁵⁾ nichtionischer direktziehender Farbstoff | |

### Beispiel 1: Zeitersparnis - Herstellung (2 kg Ansatzgröße)

| **Produktionsschritt** | **Zeitaufwand erfindungsgemäßes Färbemittel A** | **Zeitaufwand Vergleichsfärbemittel C** |
|---|---|---|
| Aufschmelzen der Fettkomponenten (80°C) | entfällt | 60 Min. |
| Einarbeitung Flocare^{®} DP/ES-502 | 10 Min. | entfällt |
| Lösen und Zugabe der Farbstoffe | 10 Min. | 10 Min. |
| Voremulgieren und Kühlen auf ca. 45°C | entfällt | 20 Min. |
| Zugabe Wirkstoffe und Parfum | 5 Min. | 5 Min. |
| Kühlen des Batches auf ca. 30°C | entfällt | 10 Min. |
| SUMME | 25 Min. | 105 Min. |

### Beispiel 2: Wasserersparnis - Herstellung (2 kg Ansatzgröße)

Gekühlt wurde mit fließendem Wasser. Je länger die Kühlzeiten für einen Batch sind, desto höher ist der Verbrauch an Kühlwasser und Energie.

Aus Beispiel 1 ergibt sich durch das Wegfallen der Kühlzeiten eine große Wasser- und Energieersparnis.

| | |
|---|---|
| Kühlzeit - Herstellung des erfindungsgemäßen Färbemittels A: | 0 Min. |
| Kühlzeit - Herstellung des Vergleichsfärbemittels C: | 30 Min. |

## Patentansprüche

1. Nicht-oxidatives Mittel zum nicht-permanenten Färben von keratinischen Fasern, insbesondere menschlichen Haaren, enthaltend in einem kosmetischen Träger
(a) mindestens einen direktziehenden Farbstoff in einer Gesamtmenge von 0,01 - 5 Gew.-%, bezogen auf das Gesamtgewicht des Mittels,
(b) Natriumpolyacrylat mit der CAS-Nummer 9003-04-7 mit einer massenmittleren Molmasse M_{w} im Bereich von 1.000.000 bis 20.000.000 Dalton, bevorzugt 6.000.000 bis 15.000.000 Dalton, in einer Gesamtmenge von 0,6 - 3 Gew.-%, bezogen auf das Gewicht des Mittels,
(c) 70 - 95 Gew.-% Wasser, bezogen auf das Gewicht des Färbemittels,
**dadurch gekennzeichnet, dass** weiterhin mindestens ein vernetztes Copolymer aus Acrylsäure und nicht-ethoxylierten Estern der Acrylsäure mit linearen C10-C30-Monoalkoholen (d) enthalten ist, wobei das vernetzte Copolymer (d) ausgewählt ist aus Copolymeren mit der INCI-Bezeichnung Acrylates/C10-30 Alkyl Acrylate Crosspolymer,
wobei das nicht-oxidative Färbemittel dadurch erhalten wird, dass das Natriumpolyacrylat als in einer Wasser-in-Öl-Emulsion vorgeliert eingesetzt wird, wobei besagte Wasser-in-Öl-Emulsion, jeweils bezogen auf ihr Gewicht, 40 - 60 Gew.-% Natriumpolyacrylat, insgesamt 25 - 45 Gew.-% Öl(e), insgesamt 0,5 - 4,9 Gew.-% Tensid(e), darunter 0,5 - 4,9 Gew.-% Niotensid(e), und 0,5 - 4,9 Gew.-% Wasser enthält.

2. Mittel nach Anspruch 1, **dadurch gekennzeichnet, dass** es mindestens einen nichtionischen direktziehenden Farbstoff, bevorzugt ausgewählt aus der Gruppe bestehend aus HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9, sowie 1,4-Diamino-2-nitrobenzol, 2-Amino-4-nitrophenol, 1,4-Bis-(2-hydroxyethyl)amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol oder Mischungen dieser Farbstoffe, enthält.

3. Mittel nach Anspruch 1 oder 2, **dadurch gekennzeichnet, dass** es mindestens einen anionischen direktziehenden Farbstoff, bevorzugt ausgewählt aus der Gruppe bestehend aus Acid Yellow 1, Acid Yellow 3, Acid Yellow 9, Acid Yellow 17, Acid Yellow 23, Acid Yellow 36, Acid Yellow 121, Acid Orange 6, Acid Orange 7, Acid Orange 10, Acid Orange 11, Acid Orange 15, Acid Orange 20, Acid Orange 24, Acid Red 14, Acid Red 18, Acid Red 27, Acid Red 33, Acid Red 35, Acid Red 51, Acid Red 52, Acid Red 73, Acid Red 87, Acid Red 95, Acid Red 184, Acid Red 195, Acid Violet 43, Acid Violet 49, Acid Violet 50, Acid Blue 1, Acid Blue 3, Acid Blue 7, Acid Blue 104, Acid Blue 9, Acid Blue 62, Acid Blue 74, Acid Blue 80, Acid Green 3, Acid Green 5, Acid Green 9, Acid Green 22, Acid Green 25, Acid Green 50, Acid Black 1, Acid Black 52, Food Yellow 8, Food Blue 5, D&C Yellow 7, D&C Yellow 8, D&C Orange 4, D&C Green 5, D&C Orange 10, D&C Orange 11, D&C Red 21, D&C Red 27, D&C Red 33, D&C Violet 2, D&C Brown 1 und Tetrabromphenolblau oder Mischungen dieser Farbstoffe, enthält.

4. Mittel nach einem der Ansprüche 1 bis 3, **dadurch gekennzeichnet, dass** es mindestens einen kationischen direktziehenden Farbstoff, bevorzugt Basic Blue 7, Basic Blue 26, Basic Violet 2, Basic Violet 14, Basic Yellow 57, Basic Red 76, Basic Blue 99, Basic Brown 16, Basic Brown 17, HC Blue 16, Basic Yellow 87, Basic Orange 31 und Basic Red 51 oder Mischungen dieser Farbstoffe, enthält.

5. Mittel nach einem der Ansprüche 1 bis 4, **dadurch gekennzeichnet, dass** mindestens ein direktziehender Farbstoff in einer Gesamtmenge von 0,1 - 4 Gew.-%, insbesondere von 0,2 - 3,5 Gew.-%, besonders bevorzugt 0,3 - 3 Gew.-%, weiter außerordentlich bevorzugt 0,5 - 2 Gew.-%, weiter außerordentlich bevorzugt 0,7 - 1,2 Gew.-%, jeweils bezogen auf das Gesamtgewicht des Mittels, enthalten ist.

6. Mittel nach einem der Ansprüche 1 bis 5, **dadurch gekennzeichnet, dass** das Natriumpolyacrylat in einer Gesamtmenge von 0,6 - 2 Gew.-%, bevorzugt von 0,6 - 1,1 Gew.-%, jeweils bezogen auf das Gewicht des Mittels, enthalten ist.

7. Mittel nach einem der Ansprüche 1 bis 6, **dadurch gekennzeichnet, dass** das mindestens eine vernetzte Copolymer aus Acrylsäure und nicht-ethoxylierten Estern der Acrylsäure mit linearen C10-C30-Monoalkoholen (d) in einer Gesamtmenge von 0,05 - 3 Gew.-%, vorzugsweise 0,1 - 1,5 Gew.-%, bevorzugt 0,2 - 1 Gew.-%, jeweils bezogen auf das Gewicht des Mittels, enthalten ist.

8. Mittel nach einem der Ansprüche 1 bis 7, **gekennzeichnet durch** eine Viskosität im Bereich von 8.000 - 100.000 mPas, vorzugsweise 10.000 - 80.000 mPas, bevorzugt 12.000 - 60.000 mPas, insbesondere 13.000 - 50.000 mPas, jeweils gemessen bei 20°C mit einem Brookfield-Rotationsviskosimeter bei einer Rotationsfrequenz von 4 min⁻¹ mit Spindel 5.

9. Mittel nach einem der Ansprüche 1 bis 8, **gekennzeichnet durch** einen pH-Wert im Bereich von 3,0 - 11, besonders bevorzugt 4,5 - 10,5, besonders bevorzugt 5,5 - 10, insbesondere 6 - 9,5, jeweils gemessen bei 22°C.

10. Mittel nach einem der Ansprüche 1 bis 9, **dadurch gekennzeichnet, dass** weiterhin mindestens eine oberflächenaktive Substanz (e), vorzugsweise ausgewählt aus der Gruppe bestehend aus anionischen Tensiden, zwitterionischen Tensiden, amphoteren Tensiden, nichtionischen Tensiden und Emulgatoren, enthalten ist.

11. Mittel nach einem der Ansprüche 1 bis 10, **dadurch gekennzeichnet, dass** weiterhin mindestens ein anionisches oder zwitterionisches Tensid enthalten ist, bevorzugt ausgewählt aus der Gruppe bestehend aus C₈-C₂₀-Alkylsulfaten, C₈-C₂₀-Alkylethersulfaten und C₈-C₂₀-Ethercarbonsäuren, jeweils mit 8 bis 20 C-Atomen in der Alkylgruppe und 0 bis 12 Ethylenoxidgruppen im Molekül, wobei Natriumlaureth(2)sulfat besonders bevorzugt ist, weiterhin aus Kokosacylaminopropyl-dimethylammoniumglycinat sowie aus Mischungen dieser Tenside, wobei bevorzugt mindestens ein Anion- oder Zwitteriontensid in einer Gesamtmenge von 0,3 - 5 Gew.-%, vorzugsweise 0,5 - 4 Gew.-%, jeweils bezogen auf das Gewicht des Mittels, enthalten ist.

12. Mittel nach einem der Ansprüche 1 bis 11, **dadurch gekennzeichnet, dass**, jeweils bezogen auf das Gewicht des Färbemittels, 78 - 91 Gew.-%, besonders bevorzugt 80 - 86 Gew.-%, Wasser enthalten ist.

13. Mittel nach einem der Ansprüche 1 bis 12, **dadurch gekennzeichnet, dass** mindestens ein Öl, bevorzugt in einer Gesamtmenge von 0,1 - 20 Gew.-%, besonders bevorzugt 0,5 - 10 Gew.-%, außerordentlich bevorzugt 1 bis 6 Gew.-%, und/oder mindestens ein C₁₄-C₃₀-Fettalkohol, der bei 20°C und 1013 mbar fest ist, bevorzugt in einer Gesamtmenge von 0,1 - 10 Gew.-%, bevorzugt 0,3 - 5 Gew.-%, besonders bevorzugt 0,5 bis 2 Gew.-%, jeweils bezogen auf das Gewicht des Färbemittels, enthalten ist.

14. Verfahren zur nicht-oxidativen, semipermanenten Färbung von keratinischen Fasern, insbesondere menschlichen Haaren, **dadurch gekennzeichnet, dass** das Mittel gemäß einem der Ansprüche 1 bis 13 direkt in Form eines Einkomponentenmittels auf die keratinischen Fasern appliziert wird, wobei das Verfahren vorzugsweise mit einer Einwirkzeit auf den keratinischen Fasern von 5 Sekunden bis 45 Minuten, bevorzugt 1 bis 40 Minuten, besonders bevorzugt 5 bis 30 Minuten, außerordentlich bevorzugt 10 - 20 Minuten, bei Raumtemperatur und/oder bei 30 - 60°C, vorzugsweise bei 32 - 50°C.

## Claims

1. Non-oxidative agent for non-permanent coloring of keratin fibers, in particular human hair, containing in a cosmetic carrier
(a) at least one direct dye in a total amount of 0.01-5 % by weight, based on the total weight of the agent,
(b) sodium polyacrylate with CAS number 9003-04-7 and a mass-average molecular weight M_{w} in the range from 1,000,000 to 20,000,000 daltons, preferably 6,000,000 to 15,000,000 daltons, in a total amount of 0.6-3 % by weight, based on the weight of the agent,
(c) 70-95 % by weight of water, based on the weight of the colorant,
**characterized in that** it further contains at least one crosslinked copolymer of acrylic acid and non-ethoxylated esters of acrylic acid with linear C10-C30 monoalcohols (d), wherein the crosslinked copolymer (d) is selected from copolymers with the INCI designation Acrylates/C10-30 Alkyl Acrylate Crosspolymer,
wherein the non-oxidative colorant is obtained by using the sodium polyacrylate as pre-gelled in a water-in-oil emulsion, said water-in-oil emulsion comprising, based on their weight, 40-60 % by weight sodium polyacrylate, a total of 25-45 % by weight oil(s), a total of 0.5-4.9 % by weight surfactant(s), including 0.5-4.9 % by weight nonionic surfactant(s), and 0.5-4.9 % by weight water.

2. Agent according to claim 1, **characterized in that** it contains at least one nonionic direct dye, preferably selected from the group consisting of HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9, as well as 1,4-diamino-2-nitrobenzene, 2-amino-4-nitrophenol, 1,4-bis-(2-hydroxyethyl)amino-2-nitrobenzene, 3-nitro-4-(2-hydroxyethyl)aminophenol, 2-(2-hydroxyethyl)amino-4,6-dinitrophenol, 4-[(2-hydroxyethyl)amino]-3-nitro-1-methylbenzene, 1-amino-4-(2-hydroxyethyl)amino-5-chloro-2-nitrobenzene, 4-amino-3-nitrophenol, 1-(2'-ureidoethyl)amino-4-nitrobenzene, 2-[(4-amino-2-nitrophenyl)amino]benzoic acid, 6-nitro-1,2,3,4-tetrahydroquinoxaline, 2-hydroxy-1,4-naphthoquinone, picramic acid and its salts, 2-amino-6-chloro-4-nitrophenol, 4-ethylamino-3-nitrobenzoic acid and 2-chloro-6-ethylamino-4-nitrophenol or mixtures of these dyes.

3. Agent according to claim 1 or 2, **characterized in that** it contains at least one anionic direct dye, preferably selected from the group consisting of Acid Yellow 1, Acid Yellow 3, Acid Yellow 9, Acid Yellow 17, Acid Yellow 23, Acid Yellow 36, Acid Yellow 121, Acid Orange 6, Acid Orange 7, Acid Orange 10, Acid Orange 11, Acid Orange 15, Acid Orange 20, Acid Orange 24, Acid Red 14, Acid Red 18, Acid Red 27, Acid Red 33, Acid Red 35, Acid Red 51, Acid Red 52, Acid Red 73, Acid Red 87, Acid Red 95, Acid Red 184, Acid Red 195, Acid Violet 43, Acid Violet 49, Acid Violet 50, Acid Blue 1, Acid Blue 3, Acid Blue 7, Acid Blue 104, Acid Blue 9, Acid Blue 62, Acid Blue 74, Acid Blue 80, Acid Green 3, Acid Green 5, Acid Green 9, Acid Green 22, Acid Green 25, Acid Green 50, Acid Black 1, Acid Black 52, Food Yellow 8, Food Blue 5, D&C Yellow 7, D&C Yellow 8, D&C Orange 4, D&C Green 5, D&C Orange 10, D&C Orange 11, D&C Red 21, D&C Red 27, D&C Red 33, D&C Violet 2, D&C Brown 1 and tetrabromophenol blue or mixtures of these dyes.

4. Agent according to one of claims 1 to 3, **characterized in that** it contains at least one cationic direct dye, preferably Basic Blue 7, Basic Blue 26, Basic Violet 2, Basic Violet 14, Basic Yellow 57, Basic Red 76, Basic Blue 99, Basic Brown 16, Basic Brown 17, HC Blue 16, Basic Yellow 87, Basic Orange 31, and Basic Red 51, or mixtures of these dyes.

5. Agent according to one of claims 1 to 4, **characterized in that** at least one direct dye is contained in a total amount of 0.1-4 % by weight, in particular 0.2-3.5 % by weight, particularly preferred 0.3-3 % by weight, further extremely preferably 0.5-2 % by weight, further extremely preferably 0.7-1.2 % by weight, in each case based on the total weight of the agent.

6. Agent according to one of claims 1 to 5, **characterized in that** the sodium polyacrylate is contained in a total amount of 0.6-2 % by weight, preferably 0.6-1.1 % by weight, in each case based on the weight of the agent.

7. Agent according to one of claims 1 to 6, **characterized in that** the at least one crosslinked copolymer of acrylic acid and non-ethoxylated esters of acrylic acid with linear C10-C30 monoalcohols (d) is contained in a total amount of 0.05-3 % by weight, preferably 0.1-1.5 % by weight, preferably 0.2-1 % by weight, in each case based on the weight of the agent.

8. Agent according to any of claims 1 to 7, **characterized by** a viscosity in the range of 8,000-100,000 mPas, preferably 10,000-80,000 mPas, more preferably 12,000-60,000 mPas, in particular 13,000-50,000 mPas, measured at 20°C with a Brookfield rotational viscometer at a rotational frequency of 4 min⁻¹ with spindle 5.

9. Agent according to one of claims 1 to 8, **characterized by** a pH value in the range of 3.0-11, particularly preferred 4.5-10.5, particularly preferred 5.5-10, in particular 6-9.5, each measured at 22°C.

10. Agent according to one of claims 1 to 9, **characterized in that** at least one surface-active substance (e), preferably selected from the group consisting of anionic surfactants, zwitterionic surfactants, amphoteric surfactants, nonionic surfactants, and emulsifiers, is also contained.

11. Agent according to one of claims 1 to 10, **characterized in that** it further contains at least one anionic or zwitterionic surfactant, preferably selected from the group consisting of C₈ -C₂₀ -alkyl sulfates, C₈ -C₂₀-alkyl ether sulfates, and C₈ -C₂₀-ether carboxylic acids, each with 8 to 20 carbon atoms in the alkyl group and 0 to 12 ethylene oxide groups in the molecule, sodium laureth(2) sulfate being particularly preferred, and furthermore from coco acylaminopropyl dimethylammonium glycinate and mixtures of these surfactants, wherein at least one anionic or zwitterionic surfactant is preferably contained in a total amount of 0.3-5 % by weight, preferably 0.5-4 % by weight, in each case based on the weight of the agent.

12. Agent according to one of claims 1 to 11, **characterized in that**, based on the weight of the dye, 78-91 % by weight, particularly preferably 80-86 % by weight, water is contained.

13. Agent according to one of claims 1 to 12, **characterized in that** at least one oil, preferably in a total amount of 0.1-20 % by weight, particularly preferably 0.5-10 % by weight, extremely preferably 1-6 % by weight, and/or at least one C₁₄-C₃₀ -fatty alcohol, which is solid at 20°C and 1013 mbar, preferably in a total amount of 0.1-10 % by weight, preferably 0.3-5 % by weight, particularly preferably 0.5-2 % by weight, in each case based on the weight of the coloring agent.

14. A method for the non-oxidative, semi-permanent coloring of keratin fibers, in particular human hair, **characterized in that** the agent according to one of claims 1 to 13 is applied directly to the keratinous fibers in the form of a single-component agent, wherein the method is preferably carried out with an exposure time on the keratinous fibers of 5 seconds to 45 minutes, preferably 1 to 40 minutes, particularly preferably 5 to 30 minutes, exceptionally preferably 10 to 20 minutes, at room temperature and/or at 30 to 60°C, preferably at 32 to 50°C.

## Revendications

1. Agent non oxydant pour la coloration non permanente des fibres kératiniques, en particulier des cheveux humains, contenant dans un support cosmétique
(a) au moins un colorant direct dans une quantité totale de 0,01 à 5 % en poids par rapport au poids total de l'agent,
(b) du polyacrylate de sodium portant le numéro CAS 9003-04-7 et ayant une masse moléculaire moyenne en poids M_{w}comprise entre 1 000 000 et 20 000 000 daltons, de préférence entre 6 000 000 et 15 000 000 daltons, en une quantité totale de 0,6 à 3 % en poids par rapport au poids du produit,
(c) 70 à 95 % en poids d'eau, par rapport au poids du colorant,
**caractérisé en ce qu'**il contient en outre au moins un copolymère réticulé d'acide acrylique et d'esters non éthoxylés de l'acide acrylique avec des monoalcools linéaires en C10-C30 (d), le copolymère réticulé (d) étant choisi parmi les copolymères portant la désignation INCI Acrylates/C10-30 Alkyl Acrylate Crosspolymer,
le colorant non oxydant étant obtenu en utilisant le polyacrylate de sodium sous forme de pré-gel dans une émulsion eau-dans-huile, ladite émulsion eau-dans-huile contenant, par rapport à son poids, 40 à 60 % en poids de polyacrylate de sodium, au total 25 à 45 % en poids% d'huile(s), au total 0,5 à 4,9 % en poids de tensioactif(s), dont 0,5 à 4,9 % en poids de niotensioactif(s), et 0,5 à 4,9 % en poids d'eau.

2. Agent selon la revendication 1, **caractérisé en ce qu'**il contient au moins un colorant non ionique à fixation directe, de préférence choisi dans le groupe constitué par HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9, ainsi que le 1,4-diamino-2-nitrobenzène, le 2-amino-4-nitrophénol, le 1,4-bis-(2-hydroxyéthyl)amino-2-nitrobenzène, 3-nitro-4-(2-hydroxyéthyl)aminophénol, 2-(2-hydroxyéthyl)amino-4,6-dinitrophénol, 4-[(2-hydroxyéthyl)amino]-3-nitro-1-méthylbenzène, 1-amino-4-(2-hydroxyéthyl)amino-5-chloro-2-nitrobenzène, 4-amino-3-nitrophénol, 1-(2'-uréidoéthyl)amino-4-nitrobenzène, 2-Acide [(4-amino-2-nitrophényl)amino]benzoïque, 6-nitro-1,2,3,4-tétrahydroquinoxaline, 2-hydroxy-1,4-naphtoquinone, acide picramique et ses sels, 2-amino-6-chloro-4-nitrophénol, acide 4-éthylamino-3-nitrobenzoïque et 2-chloro-6-éthylamino-4-nitrophénol ou des mélanges de ces colorants.

3. Agent selon la revendication 1 ou 2, **caractérisé en ce qu'**il contient au moins un colorant anionique direct, choisi de préférence dans le groupe constitué par Acid Yellow 1, Acid Yellow 3, Acid Yellow 9, Acid Yellow 17, Acid Yellow 23, Acid Yellow 36, Acid Yellow 121, Acid Orange 6, Acid Orange 7, Acid Orange 10, Acid Orange 11, Acid Orange 15, Acid Orange 20, Acid Orange 24, Acid Red 14, Acid Red 18, Acid Red 27, Acid Red 33, Acid Red 35, Acid Red 51, Acid Red 52, Acid Red 73, Acid Red 87, Acid Red 95, Acid Red 184, Acid Red 195, Acid Violet 43, Acid Violet 49, Acid Violet 50, Acid Blue 1, Acid Blue 3, Acid Blue 7, Acid Blue 104, Acid Blue 9, Acid Blue 62, Acid Blue 74, Acid Blue 80, Acid Green 3, Acid Green 5, Acid Green 9, Acid Green 22, Acid Green 25, Acid Green 50, Acid Black 1, Acid Black 52, Food Yellow 8, Food Blue 5, D&C Yellow 7, D&C Yellow 8, D&C Orange 4, D&C Green 5, D&C Orange 10, D&C Orange 11, D&C Red 21, D&C Red 27, D&C Red 33, D&C Violet 2, D&C Brown 1 et du bleu de tétrabromophénol ou des mélanges de ces colorants.

4. Agent selon l'une des revendications 1 à 3, **caractérisé en ce qu'**il contient au moins un colorant cationique de teinture directe, de préférence le bleu basique 7, le bleu basique 26, le violet basique 2, le violet basique 14, le jaune basique 57, le rouge basique 76, le bleu basique 99, le brun basique 16, le brun basique 17, HC Blue 16, Basic Yellow 87, Basic Orange 31 et Basic Red 51 ou des mélanges de ces colorants.

5. Produit selon l'une des revendications 1 à 4, **caractérisé en ce qu'**au moins un colorant direct est contenu en une quantité totale de 0,1 à 4 % en poids, en particulier de 0,2 à 3,5 % en poids, de préférence de 0,3 à 3 % en poids, de manière extrêmement préférée de 0,5 à 2 % en poids, de manière extrêmement préférée de 0,7 à 1,2 % en poids, dans chaque cas par rapport au poids total du produit.

6. Agent selon l'une des revendications 1 à 5, **caractérisé en ce que** le polyacrylate de sodium est contenu en une quantité totale de 0,6 à 2 % en poids, de préférence de 0,6 à 1,1 % en poids, dans chaque cas par rapport au poids de l'agent.

7. Agent selon l'une des revendications 1 à 6, **caractérisé en ce que** le au moins un copolymère réticulé d'acide acrylique et d'esters non éthoxylés de l'acide acrylique avec des monoalcools linéaires en C10-C30 (d) est contenu en une quantité totale de 0,05 à 3 % en poids, de préférence de 0,1 à 1,5 % en poids%, de préférence de 0,2 à 1 % en poids, dans chaque cas par rapport au poids de l'agent.

8. Agent selon l'une des revendications 1 à 7, **caractérisé par** une viscosité comprise entre 8 000 à 100 000 mPas, de préférence de 10 000 à 80 000 mPas, de préférence de 12 000 à 60 000 mPas, en particulier de 13 000 à 50 000 mPas, mesurée à chaque fois à 20 °C avec un viscosimètre rotatif Brookfield à une fréquence de rotation de 4 min⁻¹ avec une broche 5.

9. Agent selon l'une des revendications 1 à 8, **caractérisé par** un pH compris entre 3,0 et 11, de préférence entre 4,5 et 10,5, de préférence entre 5,5 et 10, en particulier entre 6 et 9,5, mesuré à 22 °C.

10. Produit selon l'une des revendications 1 à 9, **caractérisé en ce qu'**il contient en outre au moins une substance tensioactive (e), de préférence choisie dans le groupe constitué par les tensioactifs anioniques, les tensioactifs zwitterioniques, les tensioactifs amphotères, les tensioactifs non ioniques et les émulsifiants.

11. Agent selon l'une des revendications 1 à 10, **caractérisé en ce qu'**il contient en outre au moins un tensioactif anionique ou zwitterionique, choisi de préférence dans le groupe constitué par les sulfates d'alkyle en C₈-C₂₀ les sulfates d'éther d'alkyle en C₈-C₂₀ et les acides carboxyliques éther en C₈-C₂₀ -éthercarboxyliques, chacun avec 8 à 20 atomes de carbone dans le groupe alkyle et 0 à 12 groupes d'oxyde d'éthylène dans la molécule, le laureth(2)sulfate de sodium étant particulièrement préféré, ainsi que du glycinate de cocosacylaminopropyl-diméthylammonium et des mélanges de ces tensioactifs, au moins un tensioactif anionique ou zwitterionique étant de préférence contenu en une quantité totale de 0,3 à 5 % en poids, de préférence de 0,5 à 4 % en poids, par rapport au poids du produit.

12. Produit selon l'une des revendications 1 à 11, **caractérisé en ce qu'**il contient 78 à 91 % en poids, de préférence 80 à 86 % en poids, d'eau, par rapport au poids du colorant.

13. Produit selon l'une des revendications 1 à 12, **caractérisé en ce qu'**il contient au moins une huile, de préférence en une quantité totale de 0,1 à 20 % en poids, de préférence encore de 0,5 à 10 % en poids, de préférence particulièrement de 1 à 6 % en poids, et/ou au moins un alcool gras en C₁₄-C₃₀ -alcool gras, qui est solide à 20 °C et 1013 mbar, de préférence en une quantité totale de 0,1 à 10 % en poids, de préférence de 0,3 à 5 % en poids, de manière particulièrement préférée de 0,5 à 2 % en poids, dans chaque cas par rapport au poids du colorant.

14. Procédé de coloration non oxydative et semi-permanente de fibres kératiniques, en particulier de cheveux humains, **caractérisé en ce que** le produit selon l'une des revendications 1 à 13 est appliqué directement sous forme d'un produit à un composant sur les fibres kératiniques, le procédé étant de préférence mis en oeuvre avec un temps d'action sur les fibres kératiniques de 5 secondes à 45 minutes, de préférence de 1 à 40 minutes, de manière particulièrement préférée de 5 à 30 minutes, extrêmement de préférence de 10 à 20 minutes, à température ambiante et/ou à une température comprise entre 30 et 60 °C, de préférence entre 32 et 50 °C.
